# EUROPEAN PATENT APPLICATION

(11) **EP 4 365 206 A1**
(43) Date of publication of application: **08.05.2024**
(21) Application number: 22833317.5
(22) Date of filing: 30.06.2022
(51) Int. Cl.: C08C 1/00, C08J 11/26, C08L 9/00, C08L 91/00, B09B 3/40

(54) **METHOD FOR PYROLYSIS OF USED RUBBER AND RUBBER MATERIAL FOR PYROLYSIS**

(30) Priority: 30.06.2021 JP 2021109731
(71) Applicant: Bridgestone Corporation, Chuo-Ku Tokyo 104-8340 (JP)
(72) Inventor: NASHI, Takayuki, Tokyo 104-8340 (JP); HOJO, Masahiro, Tokyo 104-8340 (JP)
(74) Representative: Marks & Clerk LLP
(86) International application number: PCT/JP2022/026430
(87) International publication number: WO 2023/277171

(57) **Abstract**

Provided is a thermal decomposition method of used rubber that has excellent yields of isoprene and limonene without reducing the efficiency of thermal decomposition. The method is a method of thermally decomposing used rubber, including bringing the used rubber into contact with or swelling the used rubber in a medium, where the medium has a SP value of 8.3 (cal/cm³)^{1/2} to 11 (cal/cm³)^{1/2} and an endothermic peak temperature of 235 °C or higher measured by thermogravimetry (TGA), and the medium is liquid at 150 °C, and then performing thermal decomposition.

## Description

### TECHNICAL FIELD

This disclosure relates to a thermal decomposition method of used rubber and a rubber material for thermal decomposition.

### BACKGROUND

Polymeric materials such as rubber materials have been produced and consumed in large quantities as useful functional materials in the industrial field. Disposal of the large amounts of polymeric waste thus produced has become one of the problems that society must solve.

For example, JP 2014-237766 A (PTL 1) describes a thermal decomposition method, where a technique of thermally decomposing a used rubber such as a waste tire and recycling the material is aimed at efficiently performing thermal decomposition, the method includes an internal circulation process of circulating a pyrolysis gas and the oxygen-free gas inside a pyrolysis furnace using an internal circulation device, a heating-external circulation process is performed after the heating of the pyrolysis furnace starts and until the amount of pyrolysis gas formed reaches its maximum value, and the internal circulation process is performed after the amount of pyrolysis gas formed reaches the maximum value.

When used rubber containing an isoprene-skeleton elastomer is thermally decomposed, isoprene and limonene are obtained as components which can be reused. Further, when used rubber containing a butadiene-skeleton elastomer and a styrene-skeleton elastomer is thermally decomposed, butadiene and styrene are obtained as components which can be reused.

For the thermal decomposition of used rubber, it is desired to increase the yield of the monomer components of the elastomer as described above, in addition to the above-mentioned efficiently performing the thermal decomposition.

### CITATION LIST

### Patent Literature

PTL 1: JP 2014-237766 A

### SUMMARY

### (Technical Problem)

It could thus be helpful to provide a thermal decomposition method of used rubber that has an excellent yield of monomer components of elastomers without reducing the efficiency of thermal decomposition. Further, it could be helpful to provide a rubber material for thermal decomposition that can increase the yield of monomer components of elastomers.

### (Solution to Problem)

We have conducted studies on a method of thermally decomposing used rubber containing various elastomers to solve the above problem. As a result, we have found that, by bringing the used rubber into contact with or swelling the used rubber in a medium, where the medium is a liquid medium with a specific SP value and a specific endothermic peak temperature, as a preliminary step to thermal decomposition, it is possible to increase the yield of monomer components of elastomers without affecting the thermal decomposition.

In other words, the thermal decomposition method of used rubber of the present disclosure is a method of thermally decomposing used rubber, where the used rubber is brought into contact with or swollen in a medium and then thermally decomposed, the medium has a SP value of 8.3 (cal/cm³)^{1/2} to 11 (cal/cm³)^{1/2} and an endothermic peak temperature of 235 °C or higher measured by thermogravimetry (TGA), and the medium is liquid at 150 °C.

With the above configuration, it is possible to increase the yield of isoprene and limonene without reducing the efficiency of thermal decomposition.

The rubber material for thermal decomposition of the present disclosure contains
a rubber containing at least one type of elastomer selected from an isoprene-skeleton elastomer, a butadiene-skeleton elastomer, and a styrene-skeleton elastomer, and
a medium having a SP value of 8.3 (cal/cm³)^{1/2} to 11 (cal/cm³)^{1/2} and an endothermic peak temperature of 380 °C or higher measured by thermogravimetry (TGA), which is liquid at 150 °C.

With the above configuration, the yield of monomer components of elastomers can be increased.

### (Advantageous Effect)

According to the present disclosure, it is possible to provide a thermal decomposition method of used rubber that has an excellent yield of monomer components of elastomers without reducing the efficiency of thermal decomposition. Further, according to the present disclosure, it is possible to provide a rubber material for thermal decomposition that can increase the yield of monomer components of elastomers.

### DETAILED DESCRIPTION

The following describes embodiments of the present disclosure in detail.

### <Thermal decomposition method of used rubber>

The thermal decomposition method of used rubber of the present disclosure is a method of thermally decomposing used rubber.

As used herein, the used rubber that is subject to thermal decomposition contains an elastomer such as an isoprene-skeleton elastomer, a butadiene-skeleton elastomer, and a styrene-skeleton elastomer (hereinafter sometimes simply referred to as "elastomer").

The elastomer is, for example, an elastomer component having an isoprene unit, a butadiene unit, a styrene unit, or the like as a main skeleton, and specific examples thereof include a natural rubber (NR), a synthetic isoprene rubber (IR), a styrene-isoprene thermoplastic elastomer (SIS), a butadiene rubber (BR), and a styrene-butadiene rubber (SBR).

The elastomer may be an elastomer component other than an isoprene-skeleton elastomer, a butadiene-skeleton elastomer, and a styrene-skeleton elastomer. However, from the viewpoint of ease of use and usefulness of the recovered monomer components, it is preferably at least one type of elastomer selected from an isoprene-skeleton elastomer, a butadiene-skeleton elastomer, and a styrene-skeleton elastomer, and it more preferably contains at least an isoprene-skeleton elastomer.

The content of the elastomer in the rubber component of the used rubber is not particularly limited. It is appropriately changed depending on the type of used rubber.

The used rubber in the present disclosure refers to a rubber material that has been used, such as rubber waste. Examples thereof include tire waste such as a tire divided into 4 to 32 pieces, peeled rubber, spew, and buff powder, and rubber materials such as a rubber hose, a tube, and a conveyor belt.

The used rubber may be a vulcanized rubber or an unvulcanized rubber.

The recovered components obtained by thermally decomposing the used rubber are determined by the type of monomers contained. For example, when the above-described isoprene-skeleton elastomer is contained, isoprene and limonene are contained in the recovered components. Further, when the butadiene-skeleton elastomer is contained in the used rubber, butadiene is contained in the recovered components, and when the butadiene-skeleton elastomer is contained in the used rubber, styrene is contained in the recovered components.

As used herein, the isoprene refers to hydrocarbon having two double bonds of a structural formula of CH₂=C(CH₃)CH=CH₂. There are three types of limonene: d-limonene, l-limonene, and d/l-limonene, all of which include two isoprene units. The d-limonene and the l-limonene are represented by the following general formula (I).

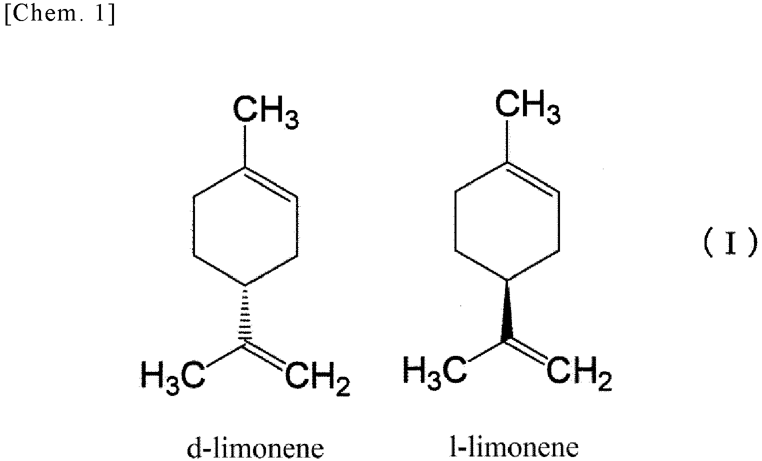

The butadiene refers to one type of unsaturated hydrocarbon having two double bonds represented by a molecular formula of C₄H₆, and examples thereof include 1,3-butadiene. The butadiene-skeleton elastomer is not particularly limited as long as it has a butadiene skeleton, and copolymers and modified butadiene rubbers having a butadiene skeleton are also included in the butadiene-skeleton elastomer.

Further, the styrene-skeleton elastomer is not particularly limited as long as it has a styrene skeleton, and copolymers and modified elastomers having a styrene skeleton are also included in the styrene-skeleton elastomer.

In the thermal decomposition method of used rubber of the present disclosure, the used rubber is brought into contact with or swollen in a medium and then thermally decomposed, where the medium has a SP value of 8.3 (cal/cm³)^{1/2} to 11 (cal/cm³)^{1/2} and an endothermic peak temperature of 235 °C or higher measured by thermogravimetry (TGA), and the medium is liquid at 150 °C.

As a preliminary step to the thermal decomposition, the used rubber is brought into contact with or swollen in a medium that has the above SP value and endothermic peak temperature and is liquid at 150 °C, and it is thermally decomposed in this state. This can increase the mobility of rubber polymer chains in the used rubber and renders the thermal decomposition easy. As a result, the yield of the monomer components of the elastomer can be increased. Further, since the contact between the used rubber and the medium and the swelling of the used rubber by the medium are performed as a preliminary step to thermal decomposition, the efficiency of the thermal decomposition process is not reduced.

The medium used for contacting or swelling the used rubber has a SP value of 8.3 (cal/cm³)^{1/2} to 11 (cal/cm³)^{1/2}. This is because when the SP value of the medium is 8.3 to 11, it is close to the SP value of the elastomer in the used rubber, which provides fluidity to the rubber and renders the thermal decomposition easy.

From the same viewpoint, the SP value of the medium is preferably 8.5 (cal/cm³)^{1/2} to 11 (cal/cm³)^{1/2}.

The SP value (solubility parameter) is measured with the Fedors method, and it can be calculated with the Fedors method described in Polymer Engineering Science, 14, 147 (1974). It is also possible to use a material whose SP value has been known.

Further, the medium used for contacting or swelling the used rubber has an endothermic peak temperature of 380 °C or higher measured by thermogravimetry (TGA). This is because it is close to the endothermic peak temperature of the elastomer in the used rubber, which provides fluidity to the rubber and renders the thermal decomposition easy.

From the same viewpoint, the endothermic peak temperature of the medium measured by TGA is preferably 235 °C or higher and more preferably 250 °C or higher.

The endothermic peak temperature measured by thermogravimetry (TGA) is the endothermic peak temperature when the heating rate is 10 °C/min or higher, preferably 10 °C/min to 20 °C/min. Regarding the measurement of TGA, the endothermic peak temperature can be obtained using a differential thermal balance (for example, "ThermoMass Photo" manufactured by Rigaku Corporation). It is also possible to use a material whose endothermic peak temperature has been known.

Further, the medium used for contacting or swelling the used rubber is liquid at 150 °C. When the medium is used together with the used rubber, the medium does not vaporize before reaching the thermal decomposition temperature and remains in a liquid state. As a result, the mobility of the rubber polymer chains in the used rubber can be increased, rendering the thermal decomposition easy. It can also be distinguished from the used rubber or a resin material which is solid at 150 °C.

Preferably, the used rubber is swollen using the medium. This can further increase the mobility of the rubber polymer chains in the used rubber, rendering the thermal decomposition easier. There are no particular limitations on the method of swelling the used rubber. For example, the used rubber is extracted with acetone, the chemical is removed, and then the used rubber is dried. Next, the used rubber is accurately weighed, and then the used rubber can be swollen by immersing it in oil (medium) for a certain period of time. The swelling ratio can be calculated with the weight before and after oil immersion.

The amount of the medium for contacting is not particularly limited, and heating may be performed if necessary. The used rubber may be brought into contact with or immersed in a heated medium, or the used rubber may be brought into contact with or immersed in the medium while performing heating.

The weight-average molecular weight (Mw) of the medium is preferably 1000 or less. In this case, the used rubber can be led to a state in which it is easy to be thermally decomposed, and the yield of the monomer components of the elastomer such as isoprene and limonene can be further increased.

From the same viewpoint, the weight-average molecular weight (Mw) of the medium is preferably 1000 or less. The weight-average molecular weight (Mw) of the medium is, for example, preferably 100 or more, more preferably 250 or more, more preferably 300 or more, and even more preferably 450 or more.

The weight-average molecular weight (Mw) of the medium can be measured, for example, by gel permeation chromatography (GPC).

Preferably, the medium has at least one oxygen atom. In this case, the used rubber can be led to a state in which it is easy to be thermally decomposed, and the yield of the monomer components of the elastomer such as isoprene and limonene can be further increased.

More preferably, the medium contains at least one selected from a fatty acid having 12 to 24 carbon atoms, a fatty acid salt, and a fatty acid ester. In this case, the used rubber can be led to a state in which it is easy to be thermally decomposed, and the yield of the monomer components of the elastomer can be further increased. Examples of such a fatty acid include myristic acid, palmitic acid, palmitoleic acid, stearic acid, oleic acid, linoleic acid, linolenic acid, and erucic acid.

Preferably, the medium has at least one ester skeleton in the molecule. In this case, the used rubber can be led to a state in which it is easy to be thermally decomposed, and the yield of the monomer components of the elastomer can be further increased. Examples of such a fatty acid ester include monoglyceride, diglyceride, triglyceride, and a fatty acid ester with aliphatic alcohol having 12 to 24 carbon atoms (wax ester).

The above-described compounds may be used alone or in combination of two or more.

The medium is a mixed oil containing an aromatic oil and asphaltene, and an oil having a SP value of 8.3 to 11 and an endothermic peak temperature of 235 °C or higher can be used.

Suitable specific examples of these media include at least one selected from a group consisting of rapeseed oil, soybean oil, olive oil, sunflower oil, safflower oil, corn oil, castor oil, jojoba oil, palm oil, palm stearin, palm olein, oleic acid, linoleic acid, asphalt-mixed naphthenic oil, a stearic acid derivative, and stearic acid glyceride. These media may be recycled or discarded ones, and may be, for example, waste cooking oil. It is also possible to use media from other sources, provided that the preferred oil has a similar composition with a fatty acid. For example, it may be an oil containing a group of compounds having a structure such as at least one selected from a fatty acid having 12 to 24 carbon atoms, a fatty acid salt, and a fatty acid ester described above.

Examples of the stearic acid derivative include stearic acid and a metal salt of stearic acid. Among the above, a metal salt of stearic acid having a melting point of 150 °C or lower is preferred. Examples of the metal of the metal salt of stearic acid include alkali metals and alkaline earth metals. Among the above, Mg, Ca, Zn, and the like are preferred, and Zn is particularly preferred.

The amount of the medium used for contacting or swelling the used rubber is not particularly limited, and it can be appropriately adjusted according to the type of the medium and the state of the used rubber.

In the thermal decomposition method of used rubber of the present disclosure, the used rubber is brought into contact with or swollen in the medium and then thermally decomposed.

There is no particular limitation on the method of thermal decomposition, and a known thermal decomposition method can be appropriately selected. For example, the thermal decomposition may be performed including a contacting process of bringing the used rubber into contact with an oxygen-free gas using a pyrolysis furnace, a furnace heating process of heating the pyrolysis furnace, a pyrolysis gas formation process of pyrolyzing the used rubber using a pyrolysis treatment device to obtain pyrolysis gas, and an oil recovery process of recovering condensed oil (oil containing the monomer components of the elastomer such as isoprene and limonene) by cooling the pyrolysis gas formed in the pyrolysis treatment device.

### <Rubber material for thermal decomposition>

The rubber material for thermal decomposition of the present disclosure contains
a rubber containing at least one type of elastomer selected from an isoprene-skeleton elastomer, a butadiene-skeleton elastomer, and a styrene-skeleton elastomer, and
a medium having a SP value of 8.3 (cal/cm³)^{1/2} to 11 (cal/cm³)^{1/2} and an endothermic peak temperature of 380 °C or higher measured by thermogravimetry (TGA), which is liquid at 150 °C.

By thermally decomposing the rubber material for thermal decomposition thus obtained, it is possible to increase the yield of monomer components of elastomers.

The structures of the isoprene-skeleton elastomer and the medium are the same as that described in the thermal decomposition method of used rubber of the present disclosure.

### EXAMPLES

The following describes the present disclosure in more detail with reference to Examples, by which the present disclosure is not intended to be limited in any way.

### <Examples 1 to 10 and Comparative Examples 1 to 10>

Under the conditions listed in Tables 1 to 3, the same amount of vulcanized rubber (containing high-cis polyisoprene rubber) was brought into contact with a medium and then thermally decomposed. The thermal decomposition was performed by heating 10 mg of a mixed sample of the vulcanized rubber and the medium at a heating rate of 20 °C/min in a helium atmosphere.

Because the medium listed in Table 1 is liquid at room temperature, thermal decomposition was performed with the vulcanized rubber swollen in the medium. Because the medium listed in Tables 2 and 3 is not liquid at room temperature, thermal decomposition was performed in a state where the vulcanized rubber was mixed with the medium.

The endothermic peak temperature (°C) measured by thermogravimetry (TGA) when the heating rate is 20 °C, the weight-average molecular weight, the SP value ((cal/cm³)^{1/2}) calculated with the Fedors method described in Polymer Engineering Science, 14, 147 (1974), and the state of swollen or not for each medium used are listed in Tables 1 to 3.

The average molecular weight of the resins of Comparative Examples 8 to 10 was measured by gel permeation chromatography (GPC) under the following conditions, and the polystyrene-equivalent weight-average molecular weight was calculated.
- Column temperature: 40 °C
- Injection volume :50 µL
- Carrier and flow rate: tetrahydrofuran 0.6 mL/min
- Sample preparation: approximately 2.5 mg of each medium was dissolved in 10 mL of tetrahydrofuran.

Further, for Comparative Examples 6 and 7 and Examples and Comparative Examples other than Example 14, the weight-average molecular weight was calculated based on the chemical structure.

### (Evaluation)

To grasp the yield of the recovered isoprene and limonene after thermal decomposition, a differential thermal balance-photoionization mass spectrometry simultaneous measuring device (manufactured by Rigaku Corporation) "Thermo Mass Photo" was used to obtain a MS ion thermogram with the data of m/z68: isoprene and m/z136: limonene, thereby measuring the peak area (A*s/mg) of isoprene and limonene in the oil content recovered after thermal decomposition.

The measured value of the peak area in each Example and each Comparative Example is listed in Tables 1 to 3 as an index value, with the measured value of the peak area in Comparative Example 1 being 100, where in Comparative Example 1, thermal decomposition was performed without contacting the vulcanized rubber with the medium. A larger index value indicates a higher yield and a better result.

### [Table 1]

**Table 1**

| | | Comparative Example 1 | Comparative Example 2 | Comparative Example 3 | Comparative Example 4 | Comparative Example 5 | Example 1 | Example 2 | Example 3 | Example 4 | Example 5 |
|---|---|---|---|---|---|---|---|---|---|---|---|
| Medium | Type | - | Silicone oil | Liquid paraffin | Dodecane | Hexadecane | Rapeseed oil | Palm stearin | Castor oil | Jojoba oil | Asphalt-mixed naphthenic oil |
| | Endothermic peak temperature (°C) | - | 657.1 | 285.5 | 128.2 | 189.3 | 431.6 | 425.4 | 406.4 | 388.8 | 448.7 |
| | Weight-average molecular weight | - | 6609.8 | 365.0 | 170.3 | 226.4 | 880.0 | 834.8 | 933.3 | 602.2 | 670 |
| | SP value ((cal/cm³)^{1/2}) | - | 7.36 | 8.06 | 7.85 | 8.01 | 8.94 | 8.94 | 10.05 | 8.60 | 9.70 |
| | Swollen or not | - | Not | Swollen | Swollen | Swollen | Swollen | Not | Not | Swollen | Not |
| Evaluation | Yield of isoprene | 100 | 78 | 94 | 162 | 151 | 910 | 915 | 850 | 994 | 479 |
| | Yield of limonene | 100 | 65 | 84 | 127 | 110 | 522 | 380 | 378 | 516 | 266 |

### [Table 2]

**Table 2**

| | | Comparative Example 1 | Comparative Example 6 | Comparative Example 7 | Example 6 | Example 7 | Example 8 | Example 9 | Example 10 |
|---|---|---|---|---|---|---|---|---|---|
| Medium | Type | - | Lauric acid | Glycerin | Zinc stearate | Stearic acid monoglyceride | Stearic acid diglyceride | Oleic acid | Linoleic acid |
| | Endothermic peak temperature (°C) | - | 210.2 | 202.2 | 401.2 | 415.3 | 422.9 | 259.2 | 246.5 |
| | Weight-average molecular weight | - | 200.3 | 92.1 | 632.3 | 358.6 | 625.0 | 282.5 | 280.5 |
| | SP value ((cal/cm³)^{1/2}) | - | 9.36 | 2002 | 9.16 | 10.76 | 9.45 | 9.15 | 9.17 |
| | Swollen or not | - | Not | Not | Not | Not | Not | Swollen | Swollen |
| Evaluation | Yield of isoprene | 100 | 94 | 86 | 426 | 473 | 884 | 687 | 641 |
| | Yield of limonene | 100 | 96 | 93 | 180 | 226 | 360 | 327 | 296 |

### [Table 3]

**Table 3**

| | | Comparative Example 1 | Comparative Example 8 | Comparative Example 9 | Comparative Example 10 |
|---|---|---|---|---|---|
| Medium | Type | - | C5 resin * | C9 resin * | Cashew-modified phenolic resin * |
| | Endothermic peak temperature (°C) | - | 418.8 | 409.8 | 435.9 |
| | Weight-average molecular weight | - | 3310 | 2050 | 8010 |
| | SP value ((cal/cm³)^{1/2}) | - | 8.07 | 9.63 | 9.43 |
| | Swollen or not | - | Not | Not | Not |
| Evaluation | Yield of isoprene | 100 | 99 | 96 | 137 |
| | Yield of limonene | 100 | 86 | 76 | 104 |

| | | | | | |
|---|---|---|---|---|---|
| * C5 resin, C9 resin, and cashew-modified phenolic resin are not liquid at 150 °C. | | | | | |

From the results in Tables 1 to 3, it can be understood that the thermal decomposition in each Example had excellent yields of isoprene and limonene. On the other hand, the thermal decomposition of each Comparative Example had a result inferior to that of Examples for both of the yields of isoprene and limonene.

### <Examples 11 to 12 and Comparative Example 11>

Under the conditions listed in Table 4, the same amount of vulcanized rubber (containing styrene-butadiene rubber (solution polymerized styrene-butadiene copolymer)) was brought into contact with a medium and then thermally decomposed. The thermal decomposition was performed by heating 10 mg of a mixed sample of the vulcanized rubber and the medium at a heating rate of 20 °C/min in a helium atmosphere.

Although the medium listed in Table 4 is liquid at room temperature, thermal decomposition was performed in a state where the vulcanized rubber was mixed with the medium without swelling the vulcanized rubber.

The endothermic peak temperature (°C) measured by thermogravimetry (TGA) when the heating rate is 20 °C, the weight-average molecular weight, the SP value ((cal/cm³)^{1/2}) calculated with the Fedors method described in Polymer Engineering Science, 14, 147 (1974), the state of swollen or not for each medium used are listed in Table 4.

### [Table 4]

**Table 4**

| | | Comparative Example 11 | Example 11 | Example 12 |
|---|---|---|---|---|
| Medium | Type | - | Rapeseed oil | Asphalt-mixed naphthenic oil |
| | Endothermic peak temperature (°C) | - | 431.6 | 448.7 |
| | Weight-average molecular weight | - | 880 | 670 |
| | SP value ((cal/cm³)^{1/2}) | - | 8.94 | 9.70 |
| | Swollen or not | - | Not | Not |
| Evaluation | Yield of styrene | 100 | 274 | 230 |
| | Yield of butadiene | 100 | 399 | 323 |

From the results in Table 4, it can be understood that the thermal decomposition in each Example had excellent yields of styrene and butadiene. On the other hand, the thermal decomposition of Comparative Example had a result inferior to that of Examples for the yield of styrene.

### INDUSTRIAL APPLICABILITY

According to the present disclosure, it is possible to provide a thermal decomposition method of used rubber that has an excellent yield of monomer components of elastomers without reducing the efficiency of thermal decomposition. Further, according to the present disclosure, it is possible to provide a rubber material for thermal decomposition that can increase the yield of monomer components of elastomers.

## Claims

1. A thermal decomposition method of used rubber, which is a method of thermally decomposing used rubber, comprising:
bringing the used rubber into contact with or swelling the used rubber in a medium, where the medium has a SP value of 8.3 (cal/cm³)^{1/2} to 11 (cal/cm³)^{1/2} and an endothermic peak temperature of 235 °C or higher measured by thermogravimetry (TGA), and the medium is liquid at 150 °C, and
then performing thermal decomposition.

2. The thermal decomposition method of used rubber according to claim 1, wherein the used rubber comprises at least one type of elastomer selected from an isoprene-skeleton elastomer, a butadiene-skeleton elastomer, and a styrene-skeleton elastomer.

3. The thermal decomposition method of used rubber according to claim 2, wherein the used rubber comprises at least an isoprene-skeleton elastomer.

4. The thermal decomposition method of used rubber according to claim 1 or 2, wherein the medium has a weight-average molecular weight (Mw) of 1000 or less.

5. The thermal decomposition method of used rubber according to claim 1 or 2, wherein the medium has at least one oxygen atom.

6. The thermal decomposition method of used rubber according to claim 5, wherein the medium comprises at least one selected from a fatty acid having 12 to 24 carbon atoms, a fatty acid salt, and a fatty acid ester.

7. The thermal decomposition method of used rubber according to claim 1 or 2, wherein the medium comprises at least one selected from a group consisting of rapeseed oil, soybean oil, olive oil, sunflower oil, safflower oil, corn oil, castor oil, jojoba oil, palm oil, palm stearin, palm olein, oleic acid, linoleic acid, a stearic acid derivative, and stearic acid glyceride.

8. A rubber material for thermal decomposition, comprising:
a rubber comprising at least one type of elastomer selected from an isoprene-skeleton elastomer, a butadiene-skeleton elastomer, and a styrene-skeleton elastomer, and
a medium having a SP value of 8.3 (cal/cm³)^{1/2} to 11 (cal/cm³)^{1/2} and an endothermic peak temperature of 380 °C or higher measured by thermogravimetry (TGA), which is liquid at 150 °C.
